# EUROPÄISCHE PATENTSCHRIFT

(11) **EP 1 377 294 B1**
(45) Veröffentlichungstag und Bekanntmachung des Hinweises auf die Patenterteilung: **22.03.2006**
(21) Anmeldenummer: 02724212.2
(22) Anmeldetag: 08.03.2002
(51) Int. Cl.: A61K 31/4515, A61K 31/137, A61P 25/16

(54) **VERWENDUNG NEUROAKTIVER SUBSTANZEN UND DEREN KOMBINATION ZUR BEHANDLUNG DER PARKINSONSCHEN KRANKHEIT**
USE OF NEUROACTIVE SUBSTANCES FOR THE TREATMENT OF PARKINSONS DISEASE AND PHARMACEUTICAL COMBINATION
UTILISATION DE SUBSTANCES NEUROACTIVES POUR TRAITER LA MALADIE DE PARKINSON ET COMBINAISON DE PRODUITS PHARMACEUTIQUES

(30) Priorität: 09.03.2001 DE 10111486
(43) Veröffentlichungstag der Anmeldung: 07.01.2004
(73) Patentinhaber: DAWIRS, Ralph, 23130 Enger (DE)
(72) Erfinder: DAWIRS, Ralph, 23130 Enger (DE)
(74) Vertreter: Schumacher & Willsau
(86) Internationale Anmeldenummer: PCT/EP2002/002571
(87) Internationale Veröffentlichungsnummer: WO 2002/072094

(56) Entgegenhaltungen:
- WO-A-01/12236
- DE-A- 2 825 322
- US-A- 5 952 501
- HUEBERT N D ET AL: "THE EFFECTS OF SOME STIMULANTS AND ANTI PSYCHOTIC DRUGS ON URINARY UNCONJUGATED TYRAMINE LEVELS IN THE RAT" RESEARCH COMMUNICATIONS IN CHEMICAL PATHOLOGY AND PHARMACOLOGY, Bd. 22, Nr. 1, 1978, Seiten 73-82, XP009000120 EN ISSN: 0034-5164
- WILLIS G.L.; SMITH G.C.; PAVEY G.M.: 'AMELIORATION OF EXPERIMENTAL PARKINSONISM BY INTRAHYPOTHALAMIC ADMINISTRATION OF HALOPERIDOL' INTERNATIONAL JOURNAL OF NEUROSCIENCE Bd. 65, Nr. 1-4, Juli 1992, GORDON AND BREACH, US, Seiten 187 - 197, XP009010517

## Beschreibung

Die Erfindung betrifft eine Verwendung neuroaktiver Substanzen zum Herstellen eines Arzneimittels beziehungsweise einer Arzneimittelkombination zur Behandlung der Parkinsonschen Krankheit. Die Erfindung betrifft weiterhin eine Arzneimittelkombination.

Das Parkinson-Syndrom ist eine der häufigsten neurologischen Erkrankungen des fortgeschrittenen Lebensalters. In der Gruppe der über 60-jährigen erkranken etwa 1%, insbesondere der Männer, am Parkinson-Syndrom. Das Parkinson-Syndrom äußert sich in zahlreichen Symptomen, welche sich grob in drei Gruppen unterteilen lassen:
1. Motorische Störungen, z.B. Rigor, Tremor;
2. Vegetative Störungen, z.B. vermehrter Speichel- und Tränenfluss, erniedrigter Blutdruck;
3. Psychische Störungen, z.B. depressive Verstimmungen, verlangsamte Denkabläufe.

Auch wenn bezüglich der Aetiologie dieser idiopathischen Erkrankung bis heute keine schlüssigen Konzepte vorliegen, ist es doch unstrittig, dass ein fortschreitendes Zugrundegehen dopaminerger Neurone der Substantia nigra eine wesentliche Grundbedingung für das spätere Auftreten der Symptome ist. Innerhalb des sogenannten "Motor-Circuit" führt die Reduktion der nigro-striären Dopaminprojektion zu einer Übergewichtung des indirekten Pfades über den Nucleus subthalamicus mit dem Ergebnis einer übermäßigen Hemmung der thalamo-kortikalen Projektionen.

Es existieren zahlreiche Konzepte zur Behandlung der Symptome der Parkinsonschen Krankheit. Dabei kommt ein ganzes Spektrum vom Medikamenten zum Einsatz. Allein oder in Kombination dienen sie der funktionellen Substitution der verlorengegangenen Dopaminzellen im Mittelhirn; hierzu gehören: L-Dopa, Dopamin-Agonisten, MAO-B-Hemmer, COMT-Hemmer, Anticholinergika, NMDA-Antagonisten;

Darüber hinaus werden die folgenden neurochirurgischen Behandlungsverfahren eingesetzt: Pallidotomie, Thalamotomie, Tiefenhirnstimulation, Transplantation Dopaminproduzierender Zellen. Diese Verfahren sind von der Natur her hochgradig invasiv und mit hohen Risiken behaftet. Auch diese Eingriffe führen letztlich zu keiner Heilung und zu keinem Aufhalten des fortschreitenden Krankheitsverlaufs. Sie können für eine beschränkte Zeit einer Entlastung der Medikamentierung dienen.

Allen Strategien des Standes der Technik ist gemeinsam, dass sie auf die Behandlung der Symptome der Parkinsonschen Krankheit gerichtet sind. Bis heute verfügt man über keine Maßnahmen zur Vorbeugung und Heilung der Parkinsonschen Krankheit.

Die Veröffentlichung von Huebert et. al. "The effects of some stimulants and anti-psychotic drugs on urinary unconjugated tyramine levels in the rat", Research communications in Chemical Pathology and Pharmacology, Bd. 22, Nr. 1 1978, Seiten 73 bis 83, betrifft den Einfluss von Antipsychotika und Stimulanzien auf den peripheren Catacholaminmetabolismus.

Die US 5,952,501 bezieht sich auf bestimmte substituierte Diazabicyclooctane, welche clozapinartige antipsychotische Wirkungsprofile zeigen. Die Substanzen werden in einem Tiermodell untersucht und bewertet. Dieses Tiermodell besteht in einer Bewertung der Fähigkeit von Antipsychotika amphetamininduzierte Hyperaktivität bei Ratten zu reduzieren.

Die DE 28 25 322 A1 betrifft alkylsubstituierte Piperidin-N-Oxide und ihre Salze, Verfahren zu ihrer Herstellung, ihre Verwendung und solche Substanzen enthaltende Arzneimittel. Es wird beschrieben, dass einige Vertreter dieser Substanzen eine antagonistische Wirkung gegenüber Haloperidol haben. Als eine mögliche Indikation für den Einsatz der Piperidin-N-Oxide wird Morbus Parkinson genannt, und es werden weiterhin die Vorzüge der Piperidin-N-Oxide und ihrer Salze zum Beispiel gegenüber Amphetamin hervorgehoben.

Der Erfindung liegt die **Aufgabe** zugrunde, die Nachteile des Standes der Technik zu beseitigen und insbesondere eine Verwendung einer oder mehrere Substanzen anzugeben, welche der Heilung beziehungsweise der Vorbeugung der Parkinsonschen Krankheit dienen.

Diese Aufgabe wird mit den Merkmalen des unabhängigen Anspruches gelöst.

Vorteilhafte Ausführungsformen und Weiterbildungen der Erfindung sind in den abhängigen Ansprüchen angegeben.

Die Erfindung besteht in einer Verwendung einer ersten und einer zweiten Substanz zum Herstellen eines Arzneimittels beziehungsweise einer Arzneimittelkombination zur Behandlung der-Parkinsonschen Krankheit, wobei als erste Substanz ein Dopamin-Antagonist vorgesehen ist, welcher postsynaptische, für einen bestimmten Neurotransmitter spezifische Rezeptoren zumindest teilweise blockiert, und als zweite Substanz ein Amphetaminderivat vorgesehen ist, welches eine Deafferentierung der auf den Neurotransmitter zurückzuführenden Innervation initiiert beziehungsweise begünstigt. Dem ersten Auftreten der Symptome der Parkinson-Krankheit geht ein sehr langer präklinischer progressiver Verlauf voran. In dieser präklinischen Phase des unerkannten fortschreitenden Verlustes nigro-striärer Projektionen leistet das dopaminerge System eine erstaunliche funktionelle Kompensation. Erst nachdem etwa 80 - 90% der dopaminergen Neurone der Substantia nigra zugrunde gegangen sind, ist diese Kompensationsfähigkeit erschöpft, und die typischen Symptome treten zutage. Insbesondere vor dem Auftreten der typischen Symptome zeigt die erfindungsgemäße Verwendung ihre besonderen Vorzüge. Durch das Blockieren der Postsynapsen wird insbesondere eine vorbereitende kompensatorische Anregung der Präsynapsen bewirkt. Anschließend kann eine Deafferentierung als "Initialzündung" für einen nachfolgenden Anstieg der Innervationsdichte dienen. Die erfindungsgemäße Verwendung ist geeignet, ein reaktives homotypisches Sprouten dopaminerger Fasern im Striatum zu induzieren, mit dem Erfolg einer altersspezifisch vermehrten Innervationsdichte dopaminerger Fasern im Striatum. Durch die Verwendung einer Substanz zum Blockieren postsynaptischer Dopaminrezeptoren kommt es zu einer kompensatorischen Anregung der Aktivität der dopaminergen Präsynapsen, so dass die Voraussetzungen für die Deafferentierung der auf den Neurotransmitter zurückzuführenden Innervation geschaffen werden. Dabei ist es besonders vorteilhaft, wenn als erste Substanz ein Dopamin-Antagonist verwendet wird. Derartige spezifische Psychopharmaka sind besonders gut geeignet, die Blockade der postsynaptischen Dopaminrezeptoren herbeizuführen. Amphetaminderivate sind in ihrer allgemeinen Wirkung auf das zentrale Nervensystem gut erforscht, so dass sich diese grundsätzlichen Kenntnisse vorteilhaft im Hinblick auf die geeignete Durchführung der Behandlung verwerten lassen. Durch Amphetaminderivate wird der oxidative Stress in Nervenzellen, insbesondere in Dopaminzellen, erhöht. Auf der Grundlage der kompensatorischen Anregung der Aktivität der dopaminergen Präsynapsen, welche eine Folge des Zuführens der ersten Substanz ist, eröffnet sich die Möglichkeit, durch die Erhöhung des oxidativen Stresses in Dopaminzellen eine partielle Deafferentierung der dopaminergen Innervation zu initiieren. Diese kann dann das reaktive homotypische Sprouten dopaminerger Fasern im Striatum induzieren.

Ebenfalls kann es vorteilhaft sein, dass als erste Substanz ein Antipsychotikum verwendet wird. Auch die Wirkung von Antipsychotika kann direkt die Funktion der Postsynapsen beeinflussen.

Besonders bevorzugt ist es, wenn als erste Substanz Haloperidol verwendet wird. Haloperidol ist ein spezifischer Dopamin-Antagonist mit besonderer Wirkung auf die D₂₋Rezeptoren, welcher im Hinblick auf seine grundsätzlichen pharmakologischen Eigenschaften gut bekannt ist. Bei geeigneter Einstellung der Dosierung lässt sich somit durch Haloperidol eine gezielte Blockade der dopaminergen Postsynapsen und folglich eine kompensatorische Anregung der Aktivität der dopaminergen Präsynapsen gezielt einstellen.

Besonders bevorzugt ist es, dass als zweite Substanz Methamphetamin verwendet wird. Mit dieser gängigen pharmakologischen Substanz lässt sich durch unterschwellige Dosierung gezielt die vorteilhafte geringfügige Deafferentierung der dopaminergen Innervation initiieren.

Besonders nützlich ist es, wenn beim bestimmungsgemäßen Gabrauch die erste Substanz vor der zweiten Substanz verwendet wird. Es kommt also zunächst zu der Blockade der postsynaptischen Dopaminrezeptoren und folglich zur kompensatorischen Anregung der Aktivität der dopaminergen Präsynapsen. Damit sind die Voraussetzungen geschaffen, dass die zweite Substanz bei geringfügiger Dosierung eine Deafferentierung der dopaminergen Innervation initiieren kann.

Vorzugsweise werden beim bestimmungsgemäßen Gebrauch die erste Substanz und/oder die zweite Substanz mehrfach verwendet. Eine mehrfache Verwendung verbessert die Wirkung des Arzneimittels.

Je nach den besonderen Bedingungen und der speziellen Zielsetzung kann es beim bestimmungsgemäßen Gebrauch bevorzugt sein, wenn die erste Substanz und/oder die zweite Substanz oral und/oder subkutan und/oder intravenös und/oder intraperitoneal verwendet werden. Die Verwendung ist also nicht auf eine spezielle Zuführung der Substanzen begrenzt; je nach Aufgabenstellung können sich aus bestimmten Formen der Zuführung jedoch spezielle Vorteile ergeben. Besonders vorteilhaft ist es jedoch, wenn die Zuführung oral erfolgt, da diese Zuführungsform beispielsweise im Hinblick auf die vorzugsweise zugeführten Substanzen Haloperidol und Methamphetamin gut bekannt sind.

Die Erfindung betrifft weiterhin eine Arzneimittelkombination zur Behandlung der Parkinsonschen Krankheit mit einem Dopamin-Antagonisten als erste Substanz, welche postsynaptische, für einen bestimmten Neurotransmitter spezifische Rezeptoren zumindest teilweise blockiert, und einem Amphetaminderivat als zweite Substanz, wobei die zweite Substanz eine Deafferentierung der auf den Neurotransmitter zurückzuführenden Innervation initiiert beziehungsweise begünstigt. Somit stehen derartige an sich bekannte Substanzen durch eine zielgerichtete Verwendung in funktioneller Einheit. Die so allgemein angegebene Arzneimittelkombination kann in vorteilhafter Weise entsprechend den vorliegend angegebenen Verwendungen ausgestaltet sein, beispielsweise dadurch, dass es sich bei der ersten Substanz um Haloperidol und bei der zweiten Substanz um Methamphetamin handelt.

Der Erfindung liegt die überraschende Erkenntnis zugrunde, dass durch den Einsatz an sich bekannter pharmakologischer Substanzen der altersbedingte Verlust dopaminerger Nervenfasern im Striatum aufgehalten werden kann. Die Innervationsdichte kann ansteigen, beziehungsweise der Abfall der Innervationsdichte kann verhindert oder verzögert werden. Grundlage der Erfindung ist, dass lokale und transsynaptische Reorganisationsprozesse des neuronalen Verschaltungsmusters natürliche Eigenschaften des Nervensystems sind, deren funktionelle Bedeutung in der normalen, nicht-invasiven, physiologischen Auseinandersetzung des Systems mit dem Milieu liegt. Neben den Hormonen scheinen insbesondere die Neurotransmitter eine wichtige Rolle bei Strukturprozessen im zentralen Nervensystem zu spielen. So nehmen die Neurotransmitter selektiv Einfluss auf das Verhalten der axonalen Wachstumskegel in der Entwicklung sowie auf progressive und regressive Aspekte struktureller Reorganisationsprozesse. Darüber hinaus sind die Neurotransmitter an Prozessen beteiligt, die den physiologischen und pathologischen Zelltod einzelner Neurone steuern. Ein fortlaufender Umbau des synaptischen Spektrums ist ganz offensichtlich für den Erhalt der funktionellen Integrität des reifen Gehirns von grundlegender Bedeutung. Mithin kann man das Nervensystem als offenes dynamisches System und somit als zwangsläufig kommunikativ-morphogenetisch verstehen, das heißt in der Offenheit für Impulse aus den Umwelten stellt sich die Ausbildung sinnvoller Strukturkorrelate für angepasstes Verhalten der Organismen als eine dem Nervensystem inhärente Eigenschaft dar. Während die Vorteile einer solchen Entwicklungsstrategie auf der Hand liegen, birgt sie jedoch auch Risiken: auch pathologische Verhaltensmuster müssen als konsequentes Ergebnis einer adaptiven, gleichwohl aberranten, Strukturentwicklung verstanden werden. In diesem Zusammenhang kommt der gezielten Anwendung der morphogenetischen Potenz neuroaktiver Substanzen (systemische Strukturpharmakologie des ZNS) in Zukunft größte Bedeutung zu. Hierzu leistet die vorliegende Erfindung bezogen auf eine verbreitete Krankheit einen wichtigen Beitrag. Insbesondere während "kritischer Perioden" der frühen Entwicklung nehmen neuroaktive Substanzen in gleicher Weise wie ihre natürlichen Vettern, etwa die Neurotransmitter, wesentlichen Einfluß auf die Neurogenese. Darüber hinaus funktionieren Neuropharmaka als indirekte Agonisten oder Antagonisten durch eine nicht-adaptive Erhöhung beziehungsweise Verringerung von Transmitteraktivitäten. Nun spielen für die Ontogenese des Gehirns insbesondere transiente neuronale Verknüpfungsmuster und funktionelle Zustände eine wichtige Rolle. Diese notwendigen, konsekutiv sich verändernden, strukturellen und funktionellen Balancen können durch Neuropharmaka nachhaltig beeinflußt werden. Unterschiedlichste Auswirkungen auf Neurogenese und Verhalten sind die Folge. Zum Beispiel unterbleibt die Ausprägung der für adulte Ratten typischen plastischen Kapazitäten zentraler DA-Rezeptoren nach fötalen Gaben von β-Endorphin. Desweiteren hat sich herausgestellt, dass fötale Gaben des DA-Rezeptor-Antagonisten Haloperidol zu einer persistierenden Down-Regulation zentraler DA-Rezeptoren führt. Darüber hinaus hat sich gezeigt, dass die chronische Gabe von Haloperidol eine dynamische Reorganisation lokaler Synapsenpopulationen, etwa in der Substantia nigra, im Striatum und im Präfrontalkortex induziert. Obwohl bezüglich der Transmitterspezifität dieser pharmakologisch induzierten Plastizität bisher keine abschließenden Angaben zu machen sind, gibt es doch konkrete Hinweise. So bewirkt etwa eine 4-monatige chronische Behandlung mit Haloperidol einen dramatischen Anstieg der Anzahl GABAerger axosomatischer Synapsen im medialen Präfrontalkortex der Ratte. Darüberhinaus gibt es Hinweise auf transsynaptisches Sprouten glutamaterger kortiko-striärer Projektionen nach 14tägiger chronischer Behandlung mit Haloperidol. Auch die Antidepressiva sind offensichtlich in der Lage, spezifische Strukturprozesse auszulösen. So induziert die chronische Behandlung mit dem Noradrenalin-reuptake-Blocker Desipramin axonales Sprouten zentraler noradrenerger und wahrscheinlich auch dopaminerger kortikaler Projektionen.

Gegenstand der Erfindung ist der gezielte Einsatz der morphogenetischen Potenz neuroaktiver Substanzen mit dem Zweck einer selektiven Reorganisation neuronaler Strukturen. Derartige Reorganisationsvorgänge werden durch den nachfolgend beschriebenen Tierversuch belegt.

Das Lebewesen, an welchem die Versuche ausgeführt wurden, ist eine Wüstenrennmaus (Meriones unguiculatus). Die Wüstenrennmaus hat ein Alter von 90 Tagen. Aufgrund ihrer Aufzucht unter stark restriktiven Bedingungen liegt im Gehirn der Maus eine herabgesetzte Innervationsdichte dopaminerger Neurone im Striatum vor, wobei die Maus allerdings noch keine typischen Parkinson-Symptome zeigt. Es liegt also ein Zustand vor, der mit einem frühen Stadium des präklinischen Verlaufs der Parkinsonschen Krankheit vergleichbar ist beziehungsweise diesem entspricht. Die Substanzen werden der Wüstenrennmaus in den nachfolgend angegebenen Dosierungen intraperitoneal zugeführt. Dabei hat sich die folgende Zuführung im Hinblick auf Menge und Abfolge an drei aufeinanderfolgenden Tagen als besonders vorteilhaft erwiesen.
Tag 1:
   09:00 Uhr 5 mg/kg Haloperidol
   13:00 Uhr 5 mg/kg Haloperidol
   17:00 Uhr 5 mg/kg Haloperidol
Tag 2:
   09:00 Uhr 5 mg/kg Haloperidol
   11:00 Uhr 1 mg/kg Methamphetamin
   13:00 Uhr 5 mg/kg Haloperidol
   17:00 Uhr 5 mg/kg Haloperidol
Tag 3:
   09:00 Uhr 5 mg/kg Haloperidol
   13:00 Uhr 5 mg/kg Haloperidol
   17:00 Uhr 5 mg/kg Haloperidol

Die konkrete beispielhaft beschriebene Ausführungsform wird in der folgenden Tabelle zusammengefasst.

| | |
|---|---|
| Tier | Wüstenrennmaus (Meriones unguiculatus) |
| Beginn der Behandlung | im Alter von 90 Tagen (jung adult) |
| Substanzen | Haloperidol in Kombination mit Methamphetamin. |
| Dosis | Haloperidol (5 mg/kg) |
| | Methamphetamin (1 mg/kg) |
| Applikation | intraperitoneal (i.p.) |
| Regime | Tag 1: |
| | 09:00 Uhr 5 mg/kg Haloperidol |
| | 13:00 Uhr 5 mg/kg Haloperidol |
| | 17:00 Uhr 5 mg/kg Haloperidol |
| | |
| | Tag 2: |
| | 09:00 Uhr 5 mg/kg Haloperidol |
| | 11:00 Uhr 1 mg/kg Methamphetamin |
| | 13:00 Uhr 5 mg/kg Haloperidol |
| | 17:00 Uhr 5 mg/kg Haloperidol |
| | |
| | Tag 3: |
| | 09:00 Uhr 5 mg/kg Haloperidol |
| | 13:00 Uhr 5 mg/kg Haloperidol |
| | 17:00 Uhr 5 mg/kg Haloperidol |

Die Versuchsergebnisse haben in signifikanter Weise eine Kompensation beziehungsweise Überkompensation des altersbedingten Abfalls der Innervationsdichte im Striatum erkennen lassen.

Die Dosierung beim Menschen erfolgt im Falle von Haloperidol beispielsweise in einem Bereich von 0,05 bis 1 mg/kg. Methamphetamin wird beispielsweise in einer Dosierung von 0,01 bis 1 mg/kg verabreicht. Es kann besonders nützlich sein, die Dosierungen von Haloperidol und Methamphetamin aufeinander abzustimmen. Das Verhältnis der Dosierungen von Haloperidol zu Methamphetamin kann in einem Bereich von 1 : 1 bis 10 : 1, vorzugsweise in einem Bereich zwischen 4 : 1 und 6 : 1 liegen.

Zusammenfassend lässt sich sagen, dass neuroaktive Substanzen in besonderer Weise geeignet sind, in die natürlichen plastischen Strukturprozesse des Gehirns einzugreifen.

## Patentansprüche

1. Verwendung einer ersten und einer zweiten Substanz zum Herstellen eines Arzneimittels beziehungsweise einer Arzneimittelkombination zur Behandlung der Parkinsonschen Krankheit, wobei
- als erste Substanz ein Dopamin-Antagonist vorgesehen ist, welcher postsynaptische, für einen bestimmten Neurotransmitter spezifische Rezeptoren zumindest teilweise blockiert, und
- als zweite Substanz ein Amphetaminderivat vorgesehen ist, welches eine Deafferentierung der auf den Neurotransmitter zurückzuführenden Innervation initiiert beziehungsweise begünstigt.

2. Verwendung nach Anspruch 1,
**dadurch gekennzeichnet,**
**dass** als erste Substanz ein Antipsychotikum zu verwenden ist.

3. Verwendung nach Anspruch 1 oder 2,
**dadurch gekennzeichnet,**
**dass** als erste Substanz Haloperidol zu verwenden ist.

4. Verwendung nach einem der vorangehenden Ansprüche,
**dadurch gekennzeichnet,**
**dass** als zweite Substanz Methamphetamin zu verwenden ist.

5. Verwendung nach einem der vorangehenden Ansprüche,
**dadurch gekennzeichnet,**
**dass** beim bestimmungsgemäßen Gebrauch die erste Substanz vor der zweiten Substanz zu verwenden ist.

6. Verwendung nach einem der vorangehenden Ansprüche,
**dadurch gekennzeichnet,**
**dass** beim bestimmungsgemäßen Gebrauch die erste Substanz und/oder die zweite Substanz mehrfach zu verwenden sind.

7. Verwendung nach einem der vorangehenden Ansprüche,
**dadurch gekennzeichnet,**
**dass** beim bestimmungsgemäßen Gebrauch die erste Substanz und/oder die zweite Substanz oral und/oder subkutan zu verwenden sind. und/oder intravenös und/oder intraperitoneal

8. Arzneimittelkombination zur Behandlung der Parkinsonschen Krankheit mit
- einem Dopamin-Antagonisten als erste Substanz, welche postsynaptische, für einen bestimmten Neurotransmitter spezifische Rezeptoren zumindest teilweise blockiert, und
- einem Amphetaminderivat als zweite Substanz, wobei die zweite Substanz eine Deafferentierung der auf den Neurotransmitter zurückzuführenden Innervation initiiert beziehungsweise begünstigt.

9. Arzneimittelkombination nach Anspruch 8,
**dadurch gekennzeichnet,**
**dass** die erste Substanz ein Antipsychotikum ist.

10. Arzneimittelkombination nach Anspruch 8 oder 9,
**dadurch gekennzeichnet,**
**dass** die erste Substanz Haloperidol ist.

11. Arzneimittelkombination nach einem der Ansprüche 8 bis 10,
**dadurch gekennzeichnet,**
**dass** die zweite Substanz Methamphetamin ist.

12. Arzneimittelkombination nach einem der Ansprüche 8 bis 11,
**dadurch gekennzeichnet,**
**dass** die erste Substanz beim bestimmungsgemäßen Gebrauch vor der zweiten Substanz zu verwenden ist.

13. Arzneimittelkombination nach einem der Ansprüche 8 bis 12,
**dadurch gekennzeichnet,**
**dass** die erste Substanz und/oder die zweite Substanz beim bestimmungsgemäßen Gebrauch mehrfach zu verwenden sind.

14. Arzneimittelkombination nach einem der Ansprüche 8 bis 13,
**dadurch gekennzeichnet,**
**dass** beim bestimmungsgemäßen Gebrauch die erste Substanz und/oder die zweite Substanz oral und/oder subkutan und/oder intravenös und/oder intraperitoneal zu verwenden sind.

15. Arzneimittelkombination nach einem der Ansprüche 8 bis 14,
**dadurch gekennzeichnet,**
**dass** sie vorbeugend auf die Symptome der Parkinsonschen Krankheit eingesetzt werden kann.

## Claims

1. Use of a first and a second substance for the production of a pharmaceutical or a pharmaceutical combination for the treatment of Parkinson's disease,
- the first substance provided being a dopamine antagonist which at least partly blocks post-synaptic receptors specific to a designated neurotransmitter,
- the second substance provided being an amphetamine derivative which initiates or promotes deafferentation of the innervation that can be attributed to the neurotransmitter.

2. Use according to Claim 1, **characterized in that** the first substance to be used is an anti-psychotic.

3. Use according to Claim 1 or 2, **characterized in that** the first substance to be used is haloperidol.

4. Use according to one of the preceding claims, **characterized in that** the second substance to be used is methamphetamine.

5. Use according to one of the preceding claims, **characterized in that**, when .used as specified, the first substance is to be used before the second substance.

6. Use according to one of the preceding claims, **characterized in that**, when used as specified, the first substance and/or the second substance are to be used repeatedly.

7. Use according to one of the preceding claims, **characterized in that**, when used as specified, the first substance and/or the second substance are to be used orally and/or subcutaneously and/or intravenously and/or intraperitoneally.

8. Pharmaceutical combination for the treatment of Parkinson's disease, having
- a dopamine antagonist as first substance, which at least partly blocks post-synaptic receptors specific to a designated neurotransmitter, and
- an amphetamine derivative as second substance, which initiates or promotes deafferentation of the innervation that can be attributed to the neurotransmitter.

9. Pharmaceutical combination according to Claim 8, **characterized in that** the first substance is an anti-psychotic.

10. Pharmaceutical combination according to Claim 8 or 9, **characterized in that** the first substance is haloperidol.

11. Pharmaceutical combination according to one of Claims 8 to 10, **characterized in that** the second substance is methamphetamine.

12. Pharmaceutical combination according to one of Claims 8 to 11, **characterized in that**, when used as specified, the first substance is to be used before the second substance.

13. Pharmaceutical combination according to one of Claims 8 to 12, **characterized in that**, when used as specified, the first substance and/or the second substance are to be used repeatedly.

14. Pharmaceutical combination according to one of Claims 8 to 13, **characterized in that**, when used as specified, the first substance and/or the second substance are to be used orally and/or subcutaneously and/or intravenously and/or intraperitoneally.

15. Pharmaceutical combination according to one of Claims 8 to 14, **characterized in that** it can be used in a preventative manner against the symptoms of Parkinson's disease.

## Revendications

1. Utilisation d'une première et d'une seconde substance afin de préparer un médicament, ou encore une combinaison de médicaments pour traiter la maladie de Parkinson, où
- un antagoniste de la dopamine est prévu en tant que première substance, qui bloque, au moins pour partie, les récepteurs postsynaptiques spécifiques d'un neurotransmetteur déterminé, et
- un dérivé d'amphétamine est prévu en tant que seconde substance, qui initie ou favorise une désafférentiation de l'innervation qui est à attribuer ou à imputer aux neurotransmetteurs.

2. Utilisation selon la revendication 1, **caractérisée en ce qu'**il convient d'utiliser un anti-psychotique en tant que première substance.

3. Utilisation selon la revendication 1 ou 2, **caractérisée en ce qu'**il convient d'utiliser de l'halopéridol en tant que première substance.

4. Utilisation selon l'une des revendications précédentes, **caractérisée en ce qu'**il convient d'utiliser une méthamphétamine en tant que seconde substance.

5. Utilisation selon l'une des revendications précédentes, **caractérisée en ce que** le bon usage prévoit d'utiliser la première substance avant la seconde substance.

6. Utilisation selon l'une des revendications précédentes, **caractérisée en ce que** le bon usage prévoit d'utiliser la première substance et/ou la seconde substance de façon répétée ou à plusieurs reprises.

7. Utilisation selon l'une des revendications précédentes, **caractérisée en ce que** le bon usage prévoit d'utiliser la première substance et/ou la seconde substance par voie orale, et/ou sous-cutanée etlou intraveineuse et/ou intrapéritonéale.

8. Combinaison de médicaments pour traiter la maladie de Parkinson, comprenant
- un antagoniste de la dopamine en tant que première substance, qui bloque, au moins pour partie, les récepteurs postsynaptiques spécifiques d'un neurotransmetteur déterminé, et
- un dérivé d'amphétamine en tant que seconde substance, qui initie ou encore favorise une désafférentiation de l'innervation qui est à attribuer ou à imputer aux neurotransmetteurs.

9. Combinaison de médicaments selon la revendication 8, **caractérisée en ce qu'**un anti-psychotique est utilisé en tant que première substance.

10. Combinaison de produits pharmaceutiques selon la revendication 8 ou 9, **caractérisée en ce que** la première substance est l'halopéridol.

11. Combinaison de produits pharmaceutiques selon l'une des revendications 8 à 10, **caractérisée en ce que** la seconde substance est la méthamphétamine.

12. Combinaison de médicaments selon l'une des revendications 8 à 11, **caractérisée en ce que** le bon usage prévoit d'utiliser la première substance avant la seconde substance.

13. Combinaison de médicaments selon l'une des revendications 8 à 12, **caractérisée en ce que** le bon usage prévoit d'utiliser la première substance et/ou la seconde substance de façon répétée ou à plusieurs reprises.

14. Combinaison de médicaments selon l'une des revendications 8 à 13, **caractérisée en ce que** le bon usage prévoit d'utiliser la première substance et/ou la seconde substance par voie orale et/ou sous-cutanée et/ou intraveineuse et/ou intrapéritonéale.

15. Combinaison de médicaments selon l'une des revendications 8 à 14; **caractérisée en ce qu'**elle peut être utilisée à titre préventif contre les symptômes de la maladie de Parkinson.
